# FASCICULE DE BREVET EUROPEEN

(11) **EP 4 537 879 B1**
(45) Date de publication et mention de la délivrance du brevet: **25.02.2026**
(21) Numéro de dépôt: 24200160.0
(22) Date de dépôt: 13.09.2024
(51) Int. Cl.: A61M 16/00, A61M 16/08, A61M 16/12, A61B 5/097

(54) **APPAREIL DE DÉLIVRANCE DE NO COMPRENANT UNE LIGNE D'ANALYSE À POMPE PIÉZOÉLECTRIQUE**
NO-ABGABEVORRICHTUNG MIT EINER ANALYSELEITUNG MIT EINER PIEZOELEKTRISCHEN PUMPE
NO DELIVERY APPARATUS INCLUDING AN ANALYSIS LINE WITH A PIEZOELECTRIC PUMP

(30) Priorité: 11.10.2023 FR 2310861
(43) Date de publication de la demande: 16.04.2025
(73) Titulaire: INOSYSTEMS, 92160 Antony (FR)
(72) Inventeur: BOULANGER, Thierry, 92160 Antony (FR); MARCHAL, Frederic, 92160 Antony (FR); SCHMITT, Mary, 92160 Antony (FR)
(74) Mandataire: Air Liquide

(56) Documents cités:
- EP-A1- 2 522 384
- WO-A1-2015/127085
- WO-A1-2022/002816
- WO-A2-2022/002824
- US-A1- 2014 275 857
- US-A1- 2017 348 503
- US-A1- 2022 296 845

## Description

L'invention porte sur un dispositif ou appareil de délivrance de NO, c'est-à-dire un appareil de fourniture d'un gaz contenant du NO, en particulier destiné à être raccordé entre une source de NO gazeux, telle une bouteille de NO sous pression, et le circuit ventilatoire ou circuit patient alimenté en gaz par un ventilateur médical, lequel appareil comprend des moyens analyseur de gaz destinés à être reliés fluidiquement au circuit patient, typiquement un appareil de délivrance de NO comprenant une ligne d'analyse à pompe piézoélectrique comprenant une pompe aspirante piézoélectrique.

Le monoxyde d'azote ou NO, lorsqu'il est inhalé, dilate les vaisseaux pulmonaires et augmente l'oxygénation en améliorant les échanges gazeux. Ces propriétés sont utilisées pour traiter différentes conditions médicales, comme décrit notamment par EP-A-560928, EP-A-1516639 et US-A-10,201,564.

Usuellement, une faible quantité de NO gazeux (i.e. quelques ppm vol.), dilué dans de l'azote (N₂) est diluée dans un flux gazeux contenant de l'oxygène, tel un mélange N₂/O₂ ou de l'air, voire de l'oxygène pur, qui est véhiculé par le circuit patient d'une installation de fourniture de gaz, et le mélange gazeux final ainsi obtenu contenant du NO et de l'oxygène et de l'azote est ensuite inhalé par le patient.

Pour ce faire, le circuit patient est relié fluidiquement à un dispositif de délivrance de NO fournissant le flux gazeux à base de NO et par ailleurs à un ventilateur médical, fournissant le flux gazeux à base d'oxygène, comme décrit par US-A-5,558,083.

Le NO étant un agent thérapeutique efficace à très faible concentration (i.e. quelques ppmv ou dizaines de ppmv), il est primordial de s'assurer que son juste dosage dans le flux gazeux final NO/N₂/O₂ pour pouvoir, si besoin est, adapter la posologie en NO en fonction de l'état du patient, i.e. amélioration ou aggravation.

A l'inverse, il est aussi important de s'assurer que le flux gazeux final NO/N₂/O₂ ne contient pas ou qu'une quantité négligeable d'espèces toxiques, en particulier de NO₂ résultant d'une oxydation d'une partie du NO par de l'O₂.

Dès lors, pour garantir une sécurité accrue du patient, l'appareil de délivrance de NO dispose généralement d'une ligne de prélèvement de gaz permettant de prélever une portion du mélange gazeux final destiné au patient, typiquement des échantillons de gaz de l'ordre de 250 ml/min, au sein du circuit respiratoire véhiculant le flux gazeux final NO/N₂/O₂, comme décrit par US2006/207594. Ces échantillons gazeux sont ensuite analysés au sein de l'appareil de délivrance de NO pour vérifier si la composition du flux gazeux final NO/N₂/O₂ correspond à celle souhaitée, en particulier pour s'assurer que:
- la concentration en NO est la plus proche possible de la posologie désirée, par exemple de l'ordre de 10 à 20 ppmv, et
- la concentration NO₂ est la plus faible possible, typiquement de 0 à 5 ppmv, car le NO₂ est un gaz toxique pouvant entraîner de graves lésions chez le patient, même à faible concentration.

Actuellement, le prélèvement du gaz dans le circuit patient est réalisé au moyen d'une pompe diaphragmatique aussi appelée pompe à diaphragme, telle que la pompe de référence série 2002 disponible auprès de la société Thomas ou celle référencée V/P 200 disponible auprès de la société Xavitech.

Les pompes diaphragmatiques comprennent généralement 2 membranes souples, aussi appelées diaphragmes, montées en regard l'une de l'autre et mis en oscillation, c'est-à-dire se rapprochant et s'éloignant l'une de l'autre à une fréquence donnée. Lors de la phase d'éloignement, un phénomène de succion s'opère et une portion du gaz à analyser vient remplir le volume séparant les deux membranes ainsi créé, alors que, lors de la phase de rapprochement, le même gaz à analyser est expulsé de la pompe. En contrôlant la fréquence de ces oscillations, typiquement entre 0 et 50 Hz, il est possible de prélever un débit « constant » de gaz à analyser, par exemple 250 ml/min.

Toutefois, en pratique, l'utilisation d'une pompe diaphragmatique pose problème pour plusieurs raisons.

Premièrement, leur mode de fonctionnement implique que le débit prélevé est lui-même par nature oscillant autour d'une valeur moyenne, par exemple 250 ml/min. Or, ces oscillations affectent les performances du ventilateur, en particulier sa capacité à détecter les « inspirations » d'un patient nouveau-né. En effet, les patients nouveau-nés ayant un appel ou « drive » respiratoire faible, c'est-à-dire produisant des efforts inspiratoires faibles et mobilisant des volumes inspiratoires également faible, de quelques ml, les ventilateurs utilisent pour cette population un capteur de débit proximal, c'est-à-dire un capteur mesurant les débits inhalés et expirés par le patient directement à sa bouche, par exemple en sortie de la sonde d'intubation. Ce capteur de débit sert d'une part à contrôler et/ou mesurer les débits inspirés par le patient pendant une phase inspiratoire, mais aussi détecter un début d'inspiration et déclencher la délivrance d'un volume afin d'assister ledit patient.

Or, les oscillations créées par le processus de prélèvement du gaz par la pompe diaphragmatique se retrouvent également au niveau du capteur de débit, ce qui perturbe le ventilateur et entraine ce que l'on appelle un auto-déclenchement, c'est-à-dire l'appréciation à tort que le patient a amorcé une inspiration et avec pour conséquence l'administration inappropriée d'un volume audit patient. De tels auto-déclenchements introduisent une asynchronie détrimentale entre le patient et le ventilateur.

Pour y remédier, les utilisateurs doivent « renforcer » le mécanisme de détection des inspirations en rendant cette détection moins sensible. Or, ceci a pour effet de demander au patient de produire un effort inspiratoire supplémentaire pour pouvoir « déclencher », de la part du ventilateur, la délivrance d'un volume.

Par ailleurs, les oscillations créées pas les pompes diaphragmatiques entrainent une pollution sonore non négligeable car les fréquences d'oscillations sont dans le spectre audible des patients. Le bruit créé par de telles pompes est de l'ordre de 50 décibels (dB), ce qui nuit fortement aux cycles de sommeil des patients et affecte potentiellement leur rétablissement.

On connait par ailleurs WO2015127085, EP2522384 et US20170348503 qui enseignent des appareils de délivrance de NO comprenant une ligne d'analyse du gaz à base de NO permettant de s'assurer que sa composition correspondant bien à celle désirée et ne contient pas de composés néfastes pour le patient, comme le NO₂. Ces appareils mettent en œuvre des pompes aspirantes classiques.

Partant de là, un problème est de limiter les oscillations dans le débit de prélèvement afin d'optimiser la détection d'inspiration par le ventilateur, et de limiter le bruit sonore généré par le processus de prélèvement de gaz.

Une solution selon l'invention concerne un appareil ou dispositif de délivrance de NO comprenant :
- un circuit principal de gaz pour véhiculer un gaz contenant du NO, en particulier un mélange NO/N₂,
- une ligne d'analyse comprenant des moyens de mesure de NO/NO₂ configurés pour opérer des mesures de concentration en NO et/ou en NO₂ au sein de ladite ligne d'analyse, et
- des moyens de pilotage reliés auxdits moyens de mesure de NO/NO₂, configurés pour traiter les mesures de concentration en NO et/ou en NO₂ opérées par lesdits moyens de mesure de NO/NO₂.

De plus, selon l'invention :
- la ligne d'analyse de l'appareil comprend des moyens (i.e. un dispositif) d'aspiration piézoélectrique pilotés par les moyens de pilotage pour aspirer du gaz à un débit d'aspiration donné,
- les moyens de pilotage sont en outre configurés pour piloter des moyens de génération de champ électrique configurés pour générer un champ électrique à une fréquence d'excitation d'au moins 20 kHz environ,
- les moyens d'aspiration piézoélectrique comprennent une pompe piézoélectrique comprenant au moins un matériau piézoélectrique choisi parmi les matériaux aptes à se déformer proportionnellement à un champ électrique appliqué audit matériau piézoélectrique, et
- ledit matériau piézoélectrique est soumis audit champ électrique généré par les moyens de génération de champ électrique de manière à se dilater ou se rétracter sous l'effet du champ électrique appliqué, en engendrant un phénomène de succion ou d'expulsion de gaz par la pompe piézoélectrique.

Selon le mode de réalisation considéré, l'appareil de l'invention peut comprendre l'une ou plusieurs des caractéristiques suivantes :
- les moyens de pilotage sont configurés pour piloter les moyens d'aspiration piézoélectrique pour aspirer du gaz à un débit d'aspiration donné inférieur ou égal à 400 mL/min, de préférence d'au moins 50 mL/min, de préférence entre 100 et 350 ml/Min, de préférence encore entre 200 et 300 mL/min.
- le débit d'aspiration donné est par exemple de l'ordre de 250 mL/min.
- les moyens de pilotage sont configurés pour piloter des moyens de génération de champ électrique configurés pour générer un champ électrique à une fréquence d'excitation entre 20 et 30 kHz environ.
- la surface de la membrane piézoélectrique est d'au moins 30 mm².
- les moyens de mesure de NO/NO₂ comprennent un capteur de NO et un capteur de NO₂.
- les moyens (ou le dispositif) d'aspiration piézoélectrique comprennent une pompe aspirante piézoélectrique exploitant lors de son fonctionnement, l'effet piézoélectrique en dynamique résultant de l'excitation fréquentielle.
- la pompe piézoélectrique comprend au moins un matériau piézoélectrique ayant une structure cristalline ou céramique.
- le matériau piézoélectrique est choisi parmi les matériaux aptes à se déformer proportionnellement sous l'effet du champ électrique qui lui est appliqué.
- le matériau piézoélectrique comprend au moins un métal.
- le matériau piézoélectrique comprend du titanate-zirconate de plomb (PZT).
- la membrane piézoélectrique est alimentée en courant électrique, en particulier en courant alternatif
- la génération de champ électrique est obtenue par variation dynamique du courant électrique appliqué traversant la membrane piézoélectrique.
- les moyens de génération de champ électrique sont pilotés par les moyens de pilotage.
- les moyens de génération de champ électrique comprennent un convertisseur de courant électrique, aussi appelé onduleur.
- le convertisseur de courant est configuré pour convertir un courant continu en courant alternatif à fréquence donnée.
- les moyens de pilotage contrôlent la valeur de la fréquence de variation du courant alternatif.
- les moyens de génération de champ électrique sont agencés dans l'appareil.
- les moyens de génération de champ électrique sont alimentés en courant électrique, en particulier en un courant continu.
- ledit au moins un matériau piézoélectrique est déposé en couche, de préférence en couche mince, sur un substrat métallique pour former une membrane piézoélectrique ayant une surface d'au moins 30 mm².
- l'épaisseur de la couche de matériau piézoélectrique, de préférence une couche mince, est comprise entre plusieurs dizaines et plusieurs centaines de µm.
- le substrat métallique comprenant du titane ou de l'aluminium.
- ledit au moins un matériau piézoélectrique a une forme de disque ou analogue.
- ledit au moins un matériau piézoélectrique est disposé dans une enceinte fermée comprenant un orifice ou port d'entrée et un orifice ou port de sortie.

De plus, selon le mode de réalisation considéré, l'appareil de délivrance de NO de l'invention peut comprendre l'une ou plusieurs des caractéristiques suivantes :
- les moyens de mesure de NO/NO₂ comprennent un premier capteur MOS à oxyde métallique (Metal Oxide Semiconductor) configuré pour déterminer la concentration en NO et un second capteur MOS à oxyde métallique configuré pour déterminer la concentration en NO₂, ou alternativement, un capteur MOS combiné configuré pour déterminer les concentrations en NO et en NO₂.
- chaque capteur MOS comprend une couche sensible comprenant au moins un oxyde métallique, une piste résistive soumise à potentiel électrique et une paire d'électrodes, par exemple un oxyde métallique choisi parmi le dioxyde de titane (TiO₂) et le dioxyde d'étain (SnO₂).
- selon un autre mode de réalisation, les moyens de mesure de NO/NO₂ comprennent des capteurs électrochimiques.
- il comprend des moyens d'alimentation électrique fournissant du courant électrique aux différents éléments ou composants de l'appareil en ayant besoin pour fonctionner, par exemple aux moyens de pilotage, aux capteurs, à la pompe etc.... De préférence, les moyens d'alimentation électrique comprennent un raccordement au secteur (110/220V) et/ou une batterie rechargeable ou autre, et éventuellement un convertisseur de courant.
- les électrodes sont reliées électriquement aux moyens de pilotage.
- les moyens de pilotage sont configurées pour déterminer une concentration en NO et/ou NO₂.
- les moyens de mesure comprennent en outre un capteur d'O₂.
- il comprend en outre un capteur de débit agencé sur la ligne d'analyse.
- il comprend un afficheur d'informations, commandé par les moyens de pilotage, pour afficher (au moins) les concentrations, i.e. teneurs, en NO et NO₂, et éventuellement en O₂.
- l'afficheur d'informations comprend un écran, en particulier l'écran d'une IGU.
- l'afficheur d'informations comprend un écran à dalle tactile.
- l'afficheur d'informations comprend un écran en couleurs ou en noir et blanc.
- l'afficheur d'informations est configuré pour afficher des valeurs de concentrations en NO et NO₂, et éventuellement en O₂, ayant été déterminées par les moyens de pilotage.
- le circuit principal de gaz comprend des moyens de contrôle de débit pour contrôler le débit de gaz dans le circuit principal de gaz, de préférence au moins une électrovanne.
- le circuit principal de gaz comprend un ou des passages de gaz, tels des conduits de gaz ou analogues.
- le circuit principal de gaz comprend au moins une entrée de gaz par laquelle un mélange NO/N₂ peut entrer dans le circuit principal.
- le circuit principal de gaz comprend au moins une sortie de gaz par laquelle le mélange NO/N₂ peut sortir du circuit principal.
- les moyens de contrôle de débit sont commandés par les moyens de pilotage pour permettre ou empêcher toute circulation de gaz dans le circuit principal de gaz, en particulier ladite au moins une électrovanne est pilotée par les moyens de pilotage (i.e. électrovanne pilotée).
- le circuit principal de gaz comprend en outre des moyens de contrôle de pression, en particulier un dispositif détendeur de gaz.
- les moyens de pilotage comprennent au moins un microprocesseur.
- ledit au moins un microprocesseur sont agencés sur au moins une carte électronique.
- les moyens de pilotage comprennent au moins un microprocesseur mettant en œuvre au moins un algorithme.
- l'appareil de délivrance de NO comprend un boitier ou carcasse externe.
- la ligne d'analyse de gaz, le circuit principal de gaz et les moyens de pilotage sont agencés dans le boitier de l'appareil.

Par ailleurs, l'invention concerne aussi une installation d'administration de gaz à un patient, c'est-à-dire dédiée à la fourniture d'un mélange gazeux contenant du NO à un patient, i.e. une personne, en ayant besoin, comprenant :
- un appareil de délivrance de NO pour fournir un gaz à base de NO selon l'invention, tel un mélange NO/N₂,
- un ventilateur médical pour fournir un gaz à base d'oxygène, tel de l'air ou un mélange O₂/N₂,
- un circuit patient auquel sont reliés fluidiquement l'appareil de délivrance de NO et le ventilateur médical,
- et un capteur de débit proximal agencé sur le circuit patient et relié électriquement au ventilateur médical.

Selon le mode de réalisation considéré, l'installation de fourniture de gaz de l'invention peut comprendre l'une ou plusieurs des caractéristiques suivantes :
- le ventilateur médical est un appareil d'assistance ventilatoire alimentant le circuit patient en un gaz respiratoire contenant de l'oxygène, typiquement au moins 20% vol. environ d'oxygène, de préférence au moins 20% vol. environ d'oxygène, en particulier de l'air ou un mélange N₂/O₂.
- l'appareil de délivrance de NO est alimenté en NO, en particulier en un mélange NO/N₂, par une (ou plusieurs) source de gaz contenant du NO gazeux.
- la (ou les) source de gaz contient un mélange NO/N₂ contenant entre 100 et 2500 ppm en volume de NO (ppmv), le reste étant de l'azote, de préférence moins de 1500 ppmv, le reste étant de l'azote, préférentiellement moins de 1000 ppmv, le reste étant de l'azote.
- préférentiellement, la source de gaz contient un mélange NO/N₂ contenant de 250 à 900 ppm en volume de NO, le reste étant de l'azote, par exemple de l'ordre de 800 ppm en volume de NO, le reste étant de l'azote.
- la (ou les) source de gaz est une bouteille de gaz sous pression.
- le circuit patient comprend une branche inspiratoire et une branche expiratoire.
- le ventilateur médical, le circuit principal de gaz et la ligne d'analyse de l'appareil de délivrance de NO sont raccordés fluidiquement à la branche inspiratoire du circuit patient.
- la branche inspiratoire et la branche expiratoire sont raccordées au niveau d'une pièce de jonction, telle une pièce en Y.
- le circuit patient, en particulier la branche inspiratoire, alimente une interface respiratoire, telle une sonde d'intubation trachéale ou un masque respiratoire.
- le capteur de débit proximal est agencé sur le circuit patient à proximité de l'interface respiratoire et/ou de la pièce de jonction, i.e. pièce en Y.
- le capteur de débit proximal est agencé entre la pièce de jonction et l'interface respiratoire.
- un humidificateur de gaz est agencé sur la branche inspiratoire, de préférence en aval du site d'injection du NO gazeux, i.e. du mélange NO/N₂, provenant du circuit principal de gaz de l'appareil de délivrance de NO.
- le circuit patient est raccordée fluidiquement à un port de sortie du ventilateur médical de manière à récupérer et acheminer le gaz contenant de l'oxygène délivré par le ventilateur médical, tel de l'air ou un mélange N₂/O₂.
- la (ou les) bouteille de gaz sous pression contient, lorsqu'elle est pleine, un mélange gazeux NO/N₂ à une pression d'au moins 150 à 200 bar abs, voire d'au moins 250 à 300 bar abs.
- le circuit patient comprend des conduites ou tuyaux servant à acheminer du gaz.
- le circuit patient comprend un capteur de débit principal relié électriquement aux moyens de pilotage.
- la ligne d'analyse de l'appareil de délivrance de NO est raccordée fluidiquement à la branche inspiratoire du circuit patient en un site de prélèvement de gaz situé en aval du capteur de débit principal, de préférence en aval de l'humidificateur de gaz.
- le circuit principal de gaz de l'appareil de délivrance de NO est raccordé fluidiquement à la branche inspiratoire du circuit patient, en un site d'injection situé entre le capteur de débit principal et le site de prélèvement de gaz.
- le circuit principal de gaz de l'appareil de délivrance de NO est raccordé fluidiquement à la branche inspiratoire du circuit patient via une ligne d'injection de gaz.
- la ligne d'analyse de l'appareil de délivrance de NO est raccordée fluidiquement à la branche inspiratoire du circuit patient via une ligne de prélèvement de gaz.
- la ligne d'analyse de gaz de l'appareil de délivrance de NO comprend un port de sortie communiquant avec l'atmosphère ambiante.
- le raccordement de la ligne de prélèvement au circuit patient se fait au moyen d'un dispositif de raccordement, telle une pièce en T, permettant d'opérer des prélèvements de gaz au sein de la branche inspiratoire du circuit patient afin de vérifier sa composition, i.e. les concentrations en NO et NO₂.
- le dispositif de raccordement est agencé en aval du point d'injection de NO, c'est-à-dire entre le point d'injection de NO et l'interface patient, e.g. une sonde d'intubation trachéale.
- le ventilateur médical alimente le circuit patient en un gaz respiratoire contenant de l'oxygène, typiquement au moins 20% vol. environ d'oxygène, en particulier de l'air ou un mélange N₂/O₂.
- l'appareil de délivrance de NO alimente le circuit patient en NO gazeux, en particulier en un mélange NO/N₂, de manière à diluer le NO gazeux (i.e. mélange NO/N₂) dans le flux gaz respiratoire contenant de l'oxygène et obtenir un mélange gazeux final destiné à être administré au patient.
- le mélange gazeux final comprend essentiellement de l'azote (N₂), de l'oxygène (O₂) à une concentration d'au moins 20%vol. environ, et du NO à une teneur comprise entre 1 et 80 ppmv, typiquement de l'ordre de 10 à 20 ppmv correspondant à une posologie souhaitée, i.e. un mélange gazeux NO/O₂/N₂.
- le mélange gazeux final comprend éventuellement des espèces NO₂ résultant de l'oxydation d'une partie du NO.

L'invention va maintenant être mieux comprise grâce à la description détaillée suivante, faite à titre illustratif mais non limitatif, en référence aux figures annexées parmi lesquelles :
Fig. 1 est une vue schématique partielle d'une installation d'administration de gaz à un patient comprenant un appareil de délivrance de NO incluant un analyseur de gaz à pompe diaphragmatique selon l'art antérieur.
Fig. 2 Illustre les oscillations de débit générées par une pompe à diaphragme, faisant partie de l'analyseur de gaz selon l'art antérieur.
Fig. 3 est une vue schématique partielle d'une installation d'administration de gaz à un patient comprenant un appareil de délivrance de NO incluant un analyseur de gaz à pompe piézoélectrique selon la présente invention.
Fig. 4 montre le bénéfice à remplacer la pompe à diaphragme de Fig. 1 par une pompe piézoélectrique selon l'invention, comme illustré en Fig. 3.

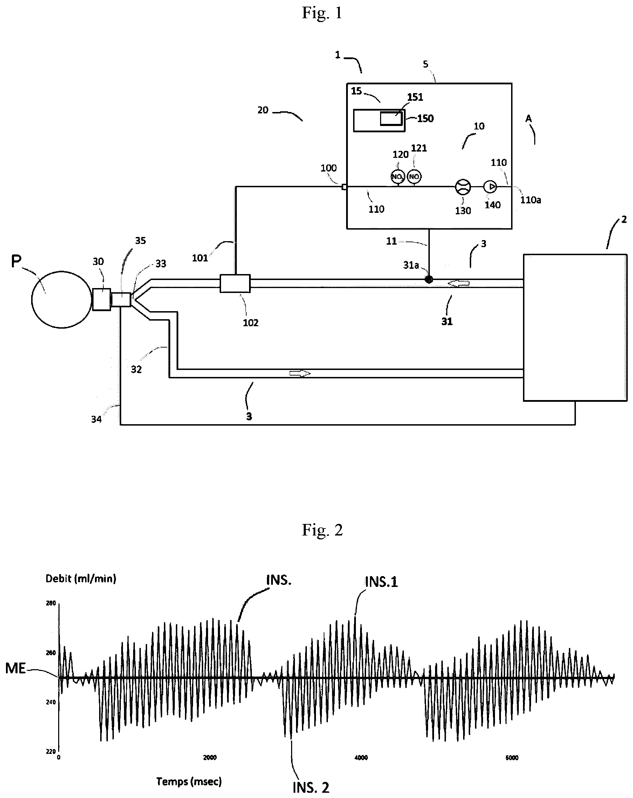

Fig. 1 schématise un mode de réalisation d'une installation de délivrance de gaz 20 servant à fournir du NO gazeux à un patient P, comprenant un appareil ou dispositif de délivrance de NO 1 pour fournir un gaz à base de NO et un ventilateur médical 2, c'est-à-dire un appareil d'assistance respiratoire délivrant un gaz respiratoire contenant de l'oxygène, typiquement au moins 20 % d'oxygène (O₂) environ, en général d'au moins 21 % d'oxygène environ.

Une telle installation 20 permet de délivrer un mélange gazeux final contenant du NO à une concentration désirée correspondant à une posologie fixée par un médecin-anesthésiste ou analogue, typiquement entre 1 et 80 ppmv de NO (i.e. ppm en volume).

Le mélange gazeux final est formé par mélange d'un flux de gaz à base de NO, tel un mélange NO/N₂ provenant de l'appareil de délivrance de NO 1 et d'un flux de gaz respiratoire contenant de l'oxygène, typiquement au moins 20 % d'oxygène (O₂) environ, tel de l'air ou un mélange O₂/N₂, provenant du ventilateur médical 2.

Le flux de gaz à base de NO est injecté (non montré) dans un circuit patient 3, en particulier dans une branche inspiratoire 31 dudit circuit patient 3, alimenté par ailleurs avec le flux de gaz contenant de l'oxygène fourni par le ventilateur médical 2.

Le débit du flux de gaz à base d'O₂ circulant dans le circuit patient 3 est mesuré par un capteur de débit principal (non montré), agencé sur le circuit patient 3 en aval du ventilateur médical 2, typiquement sur la branche inspiratoire 31. Le capteur de débit principal est relié électriquement aux moyens de pilotage 15 de l'appareil de délivrance de NO 1 afin de leur fournir des signaux ou des mesures de débit reflétant le débit du flux de gaz contenant de l'oxygène fourni par le ventilateur médical 2 et circulant dans le circuit patient 3 en direction du patient P, en particulier dans la partie amont de la branche inspiratoire 31. Le capteur de débit principal peut être un capteur de débit est du type débitmètre massique ou à différentiel de pression, ou autre.

Le mélange gazeux final obtenu par mélange des flux de NO/N₂ et d'air ou de mélange O₂/N₂ par exemple est formé essentiellement d'oxygène et d'azote, et de NO à la posologie désirée, typiquement entre 1 et 80 ppmv de NO ; il peut contenir des impuretés inévitables, par exemple de l'argon ou autres. Il est administré au patient P, pendant ses phases inspiratoires, au moyen d'une interface respiratoire 3, par exemple un masque respiratoire, une sonde d'intubation trachéale ou toute autre interface adaptée.

Les gaz expirés par le patient lors de ses phases expiratoires, sont recueillis par une branche expiratoire 32 du circuit patient 3. Les branches inspiratoire 31 et expiratoire 32 sont raccordées fluidiquement à une pièce de jonction 33, telle une pièce en Y ou similaire, laquelle est reliée par ailleurs à l'interface respiratoire 30.

L'interface respiratoire 30 permet de délivrer le gaz au patient P, en particulier pendant les phases inspiratoires, et de collecter les gaz expirés par le patient P, en particulier pendant les phases expiratoires.

Est prévu en outre un capteur de débit proximal 35 permettant de mesurer le débit administré au patient et expiré par le patient, lequel est agencé au niveau de l'interface respiratoire 30 et/ou de la pièce de jonction 33, typiquement entre elles. Le capteur de débit proximal 35 peut être de différentes technologies, tel un capteur à fil chaud, et préférentiellement adapté à la population de patients à traiter, i.e. nouveau-nés ou adultes. Ici, un câble électrique 34, relié au ventilateur médical 2, permet d'alimenter électriquement le capteur de débit proximal 35. La mesure de débit instantanée s'opère dans le ventilateur médical 2 sur la base des informations électriques retournées par le capteur de débit proximal 35. Une telle technologie est classique.

Les branches inspiratoire 31 et expiratoire 32 comprennent des conduits, canalisations, tuyaux, passages, tubulures ou similaires, par exemple des tuyaux flexibles en polymère, aptes à et configurés pour convoyer les flux gazeux.

Pendant le fonctionnement de l'installation 20, le flux de gaz circulant dans la branche inspiratoire 31 du circuit patient 3, c'est-à-dire allant du ventilateur mécanique 2 au patient P, est inhalé par le patient P, alors que les gaz expirés par ledit patient P, i.e. enrichis en CO₂, sont convoyé par la branche expiratoire 32 du circuit patient 3 jusqu'au ventilateur 2 où ils sont évacués à l'atmosphère via un orifice de mise à l'atmosphère ou analogue.

Par ailleurs, l'appareil de délivrance de NO 1 est d'architecture et de fonctionnement classiques pour ce qui est de la fourniture de NO, typiquement d'un mélange NO/N₂. Il comprend (non montrés) un circuit principal de gaz interne pour véhiculer un gaz contenant du NO, en particulier une (ou des) ligne interne d'acheminement de gaz, typiquement d'un mélange NO/N₂ comprenant de 100 à 2000 ppmv de NO (reste N₂), typiquement moins de 1000 ppmv.

Des moyens de contrôle du débit, telle une électrovanne, et/ou de la pression du flux gazeux, tel un dispositif détendeur de gaz sont agencés sur le circuit principal de gaz. Les moyens de contrôle du débit sont préférentiellement pilotés par les moyens de pilotage 15 de l'appareil de délivrance de NO 1 comprenant un ou des (micro)processeur(s) portés par une (des) carte électronique 150, à partir des signaux ou mesures de débit fournis par le capteur de débit principal. Le capteur de débit principal est avantageusement agencé sur le circuit patient 3, typiquement la branche inspiratoire 31, en amont du site d'injection 31a de la branche inspiratoire 31 du circuit patient 3 où se fait l'injection de NO (i.e. du mélange NO/azote) dans la branche inspiratoire 31 et son mélange avec le flux de gaz contenant de l'oxygène provenant du ventilateur médical 2.

Le mélange NO/N₂ comprenant par exemple de 100 à 2000 ppmv de NO (reste N₂) provient généralement d'une ou plusieurs sources de gaz, e.g. une ou des bouteilles de gaz comprimé (non montrées) contenant le mélange gazeux NO/N₂ à une pression pouvant atteindre 150 bar, voire 180 à 200 bar, ou plus.

Le dispositif de délivrance de NO 1 permet de fournir/d'injecter du NO, c'est-à-dire le mélange gazeux NO/azote, dans le circuit patient 3 afin qu'il s'y mélange avec le flux de gaz contenant de l'oxygène provenant du ventilateur médical 2, comme déjà expliqué.

Plus précisément, le mélange NO/azote est convoyé par une ligne d'injection de gaz 11, tel un conduit ou analogue, et l'injection de NO (i.e. du mélange NO/azote) est réalisée en un site d'injection 31a de la branche inspiratoire 31 du circuit patient 3 de manière à y opérer un mélange (i.e. une dilution) du flux (débit) de mélange NO/azote avec le flux (débit) de gaz contenant de l'oxygène (i.e. >20%vol. environ) provenant du ventilateur médical 2, tel de l'air ou un mélange O₂/N₂ et obtenir ainsi le mélange gazeux final NO/O₂/N₂.

Le mélange gazeux final est ensuite administré au patient P via l'interface respiratoire 30 comprend donc principalement de l'azote (N₂), de l'oxygène (O₂) à une teneur d'au moins 20%vol. environ, et du NO à une teneur comprise entre 1 et 80 ppmv, typiquement de l'ordre de 10 à 20 ppmv correspondant à une posologie souhaitée.

En effet, le monoxyde d'azote ou NO, lorsqu'il est inhalé, dilate les vaisseaux pulmonaires et augmente l'oxygénation en améliorant les échanges gazeux. Ces propriétés sont utilisées pour traiter différentes conditions médicales, comme l'Hypertension Artérielle Pulmonaire du Nouveau-né ou PPHN (pour *Persistent Pulmonary Hypertension of the Newborn*), le Syndrome de Détresse Respiratoire Aigüe ou SDRA observé principalement chez l'adulte ou encore les hypertensions pulmonaires (HP) en chirurgie cardiaque chez l'adulte ou l'enfant, comme décrit notamment par EP-A-560928, EP-A-1516639 et US-A-10,201,564.

La concentration de NO dans le mélange administré au patient, correspond à une posologie (i.e. une concentration cible) déterminée par un médecin ou analogue. En général, elle est comprise entre 1 et 80 ppm en volume (ppmv), typiquement de l'ordre de 10 à 20 ppmv, en fonction de la population traitée, i.e. nouveau-nés, enfants, adolescents ou adultes, et de la maladie à traiter.

Or, du fait de la présence d'O₂ dans le mélange gazeux final NO/O₂/N₂, une partie du NO qui s'y trouve va s'oxyder et former des espèces NO₂ toxiques.

Il est dès lors primordial de s'assurer que la concentration en NO dans le mélange gazeux final correspond bien à la posologie souhaitée par le médecin et que, par ailleurs, la quantité d'espèces NO₂ toxiques soit limitée, typiquement inférieure à quelques ppmv, en général moins de 0.5 ppmv.

Pour ce faire, le dispositif de délivrance de NO 1 de Fig.1 intègre des moyens d'analyse de gaz 10, c'est-à-dire un analyseur de gaz, permettant de monitorer, i.e. mesurer, les concentrations en NO et NO₂ dans le mélange gazeux final, comme expliqué ci-après.

Comme déjà dit, l'appareil de délivrance de NO 1 comprend par ailleurs des moyens de pilotage 15 comprenant par exemple une (des) carte de commande électronique 150 et une unité de contrôle 151 à (micro)processeur(s), typiquement un microcontrôleur ou analogue. Les moyens de pilotage 15 permettent de contrôler ou commander tous les éléments électromécaniques de l'appareil de délivrance de NO 1. Plus précisément, la carte de commande électronique 150 intègre préférentiellement l'unité de contrôle 151, i.e. un ou des microprocesseurs, et est configurée pour commander et par ailleurs analyser les signaux provenant des différents composants de l'appareil de délivrance de NO 1, tels que pompe, capteurs... y compris ceux provenant des moyens d'analyse de gaz 10.

Les moyens d'analyse de gaz 10, c'est-à-dire l'analyseur de gaz, est agencé dans le boitier 5 ou carcasse externe, par exemple en polymère, de l'appareil de délivrance de NO 1.

L'analyseur de gaz 10 comprend un port d'entrée 100 agencé du côté extérieur du boitier 5 de l'appareil de délivrance de NO 1, qui est relié fluidiquement à la branche inspiratoire 31 du circuit patient 3, via une ligne de prélèvement de gaz 101.

Le raccordement de la ligne de prélèvement 101 au circuit patient 3 se fait au moyen d'un dispositif de raccordement 102 adapté, telle une pièce en T ou analogue, permettant d'opérer des prélèvements de gaz au sein de la branche inspiratoire 31 du circuit patient 3 afin de vérifier sa composition, notamment les concentrations en espèces NO et NO₂.

Le dispositif de raccordement 102 est agencé en aval du point d'injection 31a de NO, c'est-à-dire entre le point d'injection 31a de NO et l'interface patient 30, e.g. une sonde d'intubation trachéale.

Le port d'entrée 100 de l'analyseur de gaz 10 communique fluidiquement avec une ligne d'analyse de gaz 110, agencée dans le boitier 5, au sein de laquelle sont agencés successivement, à partir du port d'entrée 100, un capteur de NO₂ 120 et un capteur de NO 121, un capteur de débit 130 et un dispositif d'aspiration de gaz 140, telle une pompe aspirante.

La ligne d'analyse de gaz 110 se termine en un port de sortie 110a relié à l'atmosphère ambiante A par lequel le gaz est rejeté à l'atmosphère après avoir transité dans la ligne d'analyse de gaz 110 et avoir été mis en contact avec notamment les capteurs de NO₂ 120 et de NO 121.

La pompe aspirante 140 permet de faire circuler le gaz en créant un débit ou flux gazeux dans la ligne d'analyse de gaz 110, comme expliqué ci-après.

L'alimentation électrique de l'appareil de délivrance de NO 1, en particulier des moyens de pilotage 15, des moyens d'analyse de gaz 10 (i.e. les capteurs 120, 121, 130), de la pompe à diaphragme 140, est assurée par une source de courant électrique et/ou des moyens d'alimentation électrique (non montrés), par exemple une liaison au courant du secteur (110/220V) de type cordon électrique et prise de raccordement, et/ou une (ou des) batterie d'alimentation électrique, de préférence rechargeable, et/ou un transformateur de courant.

Le flux de gaz à analyser qui est aspiré par la pompe et circule dans la ligne d'analyse de gaz 110, est mis en contact avec les capteurs de NO₂ 120 et de NO 121, lesquels vont alors opérer une mesure de la concentration en NO₂ et en NO du flux gazeux et fournir ces mesures aux moyens de pilotage 15 qui vont alors les restituer à l'utilisateur, tel un médecin ou un autre personnel soignant, en commandant leur affichage sur un afficheur d'informations (non montré), tel un écran, de l'interface graphique utilisateur (IGU) de l'appareil de délivrance de NO 1.

La ligne d'analyse de gaz 110 peut aussi comprendre un ou plusieurs autres capteurs, comme un capteur d'oxygène.

Bien entendu, si besoin, les moyens de pilotage 15 peuvent aussi traiter les mesures de NO et NO₂ avant de commander leur affichage, en particulier réaliser une compensation de ces valeurs pour tenir compte de facteurs environnementaux pouvant influencer les mesures, telle la pression atmosphérique, la température et/ou l'humidité, auxquels le mélange gazeux a pu être soumis.

Selon l'art antérieur, comme déjà expliqué, la pompe aspirante 140 est généralement de type à diaphragme, aussi appelée pompe diaphragmatique. Elle est commandée par les moyens de pilotage 15 afin d'aspirer, i.e. de prélever, une partie du gaz à analyser provenant du circuit patient 3. De préférence, le débit circulant dans la ligne d'analyse de gaz 110 est maintenu constant et égal à un débit cible donné, par exemple à 250 ml/min environ. Ce pilotage de la pompe à diaphragme 140 est opéré à partir des mesures de débit opérées par le capteur de débit 130 de l'analyseur 10 et comprend un ajustage, de préférence en permanence, de la commande de la pompe à diaphragme 140 dans le but d'obtenir le débit cible désiré.

Eu égard au caractère oscillatoire intrinsèque de la pompe à diaphragme 140, comme expliqué précédemment, les moyens de pilotage 15, via notamment l'unité de contrôle 151, peuvent traiter le signal provenant du capteur de débit 130 afin de « gommer » les oscillations, ce au moyen de filtres passe-bas, moyennes temporelles etc... et permet à ladite pompe à diaphragme 140 d'atteindre et de maintenir le débit cible donne, par exemple 250 mL/min.

Dans tous les cas, la pompe 140 étant de type diaphragmatique, elle induit des oscillations autour de la valeur de débit cible, par exemple 250 ml/min.

Ainsi, Fig. 2 représente une courbe du débit en fonction du temps montrant les oscillations engendrées par une pompe diaphragmatique 140 utilisée dans la ligne d'analyse de gaz 110 d'un appareil de délivrance de NO 1 selon l'art antérieur, et mesurées par le capteur de débit 130 de la ligne d'analyse de gaz 110.

Sont visibles le débit instantané INS issu de la valeur de débit instantanée transmise par le capteur de débit 130 et une valeur moyennée ME issue du traitement du signal opéré par les moyens de pilotage 15, via notamment l'unité de contrôle 151 qui contrôlent la pompe à diaphragme 140 pour atteindre et maintenir le débit de prélèvement cible. On note que cette valeur moyennée ME est bien centrée sur le débit de 250 ml/min. Par contre, il apparait clairement que le débit instantané INS. est de nature oscillante, alternant des valeurs successivement supérieures et inférieures à la valeur moyennée ME.

Par exemple, le débit maximum enregistré du débit instantané INS., INS. 1 est de l'ordre de 275 ml/min, tandis que le débit minimum enregistré du débit instantané INS., INS. 2 est de l'ordre de 225 ml/min. Ceci correspond à des variations de débit de l'ordre de 50 ml/min au cours du temps qui viennent « parasiter » la mesure du débit proximal 35 en se propageant successivement dans la ligne de prélèvement 101, la branche inspiratoire 31 du circuit patient 3 via le dispositif de raccordement 102, et le capteur de débit proximal 35, en entrainant les problèmes susmentionnés rencontrés avec les pompes à diaphragme 140 selon l'art antérieur.

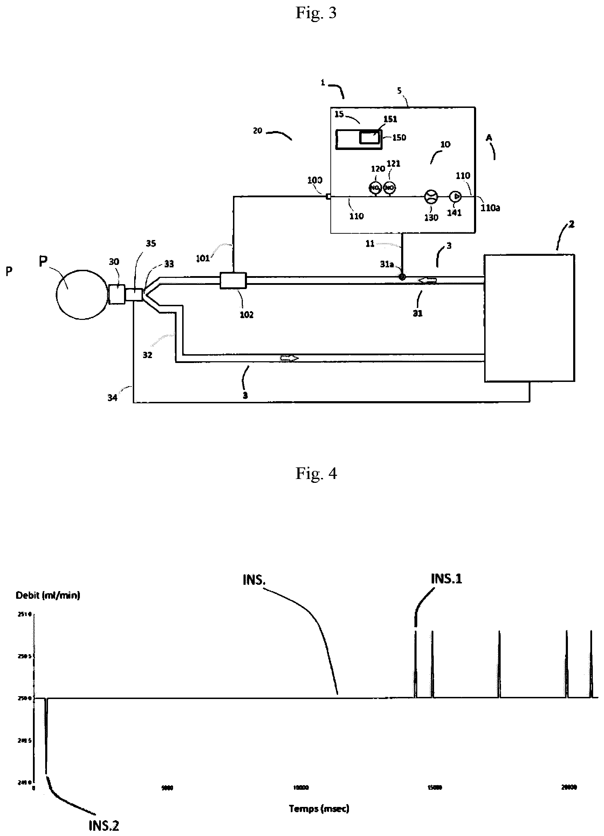

Afin de résoudre ces problèmes, selon la présente invention, on a remplacé dans l'appareil de délivrance de NO 1 de l'installation 100 de Fig. 1, la pompe à diaphragme 140 selon l'art antérieur par une pompe piézoélectrique 141, comme illustré en Fig. 3. Le reste de l'installation 100 est inchangé et son fonctionnement est identique à celui décrit en rapport avec Fig. 1.

Plus précisément, la pompe piézoélectrique 141 utilisée selon l'invention a un fonctionnement basé sur un (ou des) matériau piézoélectrique, c'est-à-dire ayant des propriétés piézoélectriques, par exemple une structure de type cristalline ou céramique, et répondant à l'excitation par un champ électrique en se déformant proportionnellement au champ électrique auquel il est soumis. Par exemple, le matériau piézoélectrique peut être du titanate zirconate de plomb qui peut se déformer à hauteur de 0.1% de ses dimensions sous l'effet d'un champ électrique. Bien entendu, le matériau piézoélectrique peut être tout autre matériau adapté.

Le matériau piézoélectrique est déposé sous forme d'une ou plusieurs couches minces de quelques dizaines à centaines de micromètres d'épaisseur sur un substrat de type métallique, par exemple du titane, de l'aluminium ou tout autre substrat adapté. On obtient alors une « membrane » mince, i.e. fine, pouvant « osciller » sous l'effet d'un champ électrique variable.

En d'autres termes, une pompe piézoélectrique 141 peut comprendre schématiquement une surface, par exemple en forme de disque ou une autre forme adaptée, comprenant un substrat adapté, par exemple métallique, et une (des) couche piézoélectrique mince déposée sur celui-ci.

Un tel ensemble piézoélectrique est placé dans une enceinte, par exemple de section circulaire, formant une cavité munie d'un port d'entrée et d'un port de sortie. Sont prévus par ailleurs des moyens de génération de champ électrique pour générer un champ électrique variable (i.e. un matériau conducteur) affectant le matériau piézoélectrique.

La membrane piézoélectrique est alimentée en courant électrique et les moyens de génération de champ électrique sont pilotés par les moyens de pilotage 15 pour opérer des variations dynamiques au fil du temps, du courant électrique fourni à la membrane piézoélectrique. Le champ électrique est alors généré par les variations dynamiques, au fil du temps, appliquées au courant électrique traversant l'ensemble piézoélectrique, c'est-à-dire la membrane piézoélectrique, en particulier un courant électrique alternatif.

Lorsque le matériau piézoélectrique se dilate ou se rétracte, du fait du champ électrique appliqué, i.e. courant électrique alternatif, un phénomène de succion ou d'expulsion s'opère par respectivement les ports d'entrée et de sortie de la pompe piézoélectrique 141. Toutefois, les faibles déformations du matériau piézoélectrique ne mobilisent qu'une quantité infime de gaz, ce qui requiert alors:
- un travail de la membrane piézoélectrique à haute fréquences pour accroitre significativement le nombre d'oscillations du matériau piézoélectrique ; et
- des dimensionnements la membrane piézoélectrique, typiquement de surface de la membrane piézoélectrique, et donc de la cavité qui la contient, qui soient suffisantes pour mobiliser « assez » de gaz par oscillation et atteindre les performances de débit escomptées.

De là, la fréquence d'excitation du matériau piézoélectrique est préférentiellement une fréquence ultrasonique, c'est-à-dire d'au moins 20 kHz, typiquement entre 20 kHz et 30 kHz.

Comme illustré sur Fig. 3, les moyens de pilotage 15 commandent la pompe piézoélectrique 141 afin d'aspirer, i.e. de prélever, une partie du gaz à analyser provenant du circuit patient 3 et transitant par la ligne de prélèvement 101 qui est reliée fluidiquement à la branche inspiratoire 31.

De préférence, les moyens de pilotage 15 commandent, i.e. pilotent, la pompe piézoélectrique 141 afin que le débit circulant dans la ligne d'analyse de gaz 110 soit maintenu constant et égal à un débit cible donné, typiquement compris entre 50 et 500 ml/Min, par exemple de l'ordre de 250 ml/min environ. Ce pilotage de la pompe piézoélectrique 141 est opéré à partir des mesures de débit opérées par le capteur de débit 130 et comprend un ajustage, de préférence en permanence, de la commande de la pompe piézoélectrique 141 dans le but d'obtenir le débit cible désiré.

Lorsque la ligne de prélèvement 101 est longue, par exemple de plusieurs mètres, elle peut présenter une résistance à l'écoulement du gaz prélevé, laquelle peut affecter les performances de la pompe piézoélectrique 141.

Dès lors, aux fréquences d'opération susmentionnées, la surface de la membrane piézoélectrique peut être un facteur à prendre en compte afin d'obtenir le débit cible, par exemple un débit cible de 250 ml/min, en considérant par ailleurs la résistance opérée par la ligne de prélèvement 101.

Des essais réalisés dans le cadre de l'invention ont montré que la surface de la membrane piézoélectrique doit être d'au moins 30 mm², lorsqu'on opère à des fréquences d'excitation comprises entre 20 et 30 kHz.

Fig. 4 représente les variations du débit gazeux au sein de la ligne d'analyse de gaz 110 lorsque l'aspiration de gaz est opérée, selon l'invention, avec une pompe piézoélectrique 141 et le débit mesuré par le capteur de débit 130 de Fig. 3.

On y voit le débit instantané INS. issu de la valeur instantanée transmise par le capteur de débit 130, centrée sur le débit cible, i.e. ici de 250 ml/min, via un contrôle adéquat de la pompe piézoélectrique 141 par les moyens de pilotage 15, typiquement l'unité de contrôle 151.

Du fait de l'utilisation d'une pompe piézoélectrique 141 en lieu et place de la pompe à diaphragme 140 selon l'art antérieur, le débit instantané INS. est parfaitement lisse, c'est-à-dire ne présentant pas d'oscillations ou présentant des oscillations extrêmement limitées.

Par exemple, sur une acquisition de 20 secondes, seuls quelques maxima et minima locaux INS.1 et INS.2 se produisent, dont la valeur ne s'écarte que peu de la valeur cible, à savoir de seulement 1 ml/min environ, c'est-à-dire plus de 50 fois plus faible que dans le cas de Fig. 2 où une pompe à diaphragme 140 selon l'art antérieur a été utilisée.

On comprend l'avantage à utiliser une pompe piézoélectrique 141 dans la ligne d'analyse de gaz 110 du ventilateur 1 selon l'invention. En effet, les variations de débit au cours du temps sont faibles et ne viennent pas « parasiter » la mesure du débit proximal 35, ce qui permet d'améliorer la sensibilité de détection d'effort inspiratoire du ventilateur médical 2 et ainsi faciliter l'effort inspiratoire des patients, notamment lorsque les patients sont des nouveau-nés.

En outre, la pompe piézoélectrique 141 présente aussi l'avantage d'être parfaitement silencieuse car les fréquences d'opération situées dans la gamme des ultrasons sont inaudibles pour l'oreille humaine.

D'une façon générale, l'installation d'administration de gaz 20 comprenant l'appareil de délivrance de NO 1 selon l'invention est adaptée à une utilisation pour traiter différentes pathologies pulmonaires, comme l'Hypertension Artérielle Pulmonaire du Nouveau-né (PPHN), le Syndrome de Détresse Respiratoire Aigüe (SDRA) et/ou les hypertensions pulmonaires (HP) en chirurgie cardiaque et ce, chez différentes populations de patient, en particulier les nouveau-nés, les adultes, les adolescents ou les enfants.

## Revendications

1. Appareil de délivrance de NO (1) comprenant :
- un circuit principal de gaz pour véhiculer un gaz contenant du NO, en particulier un mélange NO/N₂,
- une ligne d'analyse (110) comprenant des moyens de mesure de NO/NO₂ (120, 121) configurés pour opérer des mesures de concentration en NO et/ou en NO₂ au sein de ladite ligne d'analyse (110), et
- des moyens de pilotage (15) reliés auxdits moyens de mesure de NO/NO₂ (120, 121) et configurés pour traiter les mesures de concentration en NO et/ou en NO₂ opérées par lesdits moyens de mesure de NO/NO₂ (120, 121),
**caractérisé en ce que** :
- la ligne d'analyse (110) comprend des moyens d'aspiration piézoélectrique (141) pilotés par les moyens de pilotage (15) pour aspirer du gaz à un débit d'aspiration donné,
- les moyens de pilotage (15) sont en outre configurés pour piloter des moyens de génération de champ électrique configurés pour générer un champ électrique à une fréquence d'excitation d'au moins 20 kHz environ,
- les moyens d'aspiration piézoélectrique (141) comprennent une pompe piézoélectrique comprenant au moins un matériau piézoélectrique choisi parmi les matériaux aptes à se déformer proportionnellement à un champ électrique appliqué audit matériau piézoélectrique, et
- ledit matériau piézoélectrique est soumis audit champ électrique généré par les moyens de génération de champ électrique de manière à se dilater ou se rétracter sous l'effet du champ électrique appliqué, en engendrant un phénomène de succion ou d'expulsion de gaz par la pompe piézoélectrique.

2. Appareil selon la revendication 1, **caractérisé en ce que** le matériau piézoélectrique a une structure cristalline ou céramique.

3. Appareil selon la revendication 1, **caractérisé en ce que** le débit d'aspiration donné est inférieur à 400 mL/min, de préférence entre 200 et 300 mL/min.

4. Appareil selon l'une des revendications 1 ou 2, **caractérisé en ce que** les moyens de pilotage (15) sont configurés pour piloter des moyens de génération de champ électrique configurés pour générer un champ électrique à une fréquence d'excitation entre 20 et 30 kHz.

5. Appareil selon la revendication 1, **caractérisé en ce que** les moyens de mesure de NO/NO₂ (120, 121) comprennent un capteur de NO (121) et un capteur de NO₂ (120).

6. Appareil selon la revendication 1, **caractérisé en ce que** le matériau piézoélectrique comprend au moins un métal.

7. Appareil selon la revendication 6, **caractérisé en ce que** le matériau piézoélectrique comprend du titanate-zirconate de plomb (PZT).

8. Appareil selon la revendication 1, **caractérisé en ce que** les moyens d'aspiration piézoélectrique (141) comprennent une pompe aspirante piézoélectrique exploitant, pendant son fonctionnement, l'effet piézoélectrique en dynamique résultant de l'excitation fréquentielle.

9. Appareil selon l'une des revendications 1, 6 ou 7, **caractérisé en ce que** ledit au moins un matériau piézoélectrique est déposé en couche sur un substrat métallique pour former une membrane piézoélectrique ayant une surface d'au moins 30 mm².

10. Appareil selon la revendication 1, **caractérisé en ce que** les moyens de génération de champ électrique comprennent un convertisseur de courant électrique.

11. Appareil selon la revendication 10, **caractérisé en ce que** le convertisseur de courant électrique est alimenté en courant électrique continu.

12. Appareil selon la revendication 11, **caractérisé en ce que** le convertisseur de courant est configuré pour convertir le courant continu en courant alternatif à fréquence donnée.

13. Appareil selon les revendications 1 et 12, **caractérisé en ce que** les moyens de pilotage contrôlent la valeur de la fréquence de variation du courant alternatif.

14. Appareil selon la revendication 9, **caractérisé en ce que** la membrane piézoélectrique est alimentée en courant électrique alternatif.

15. Installation d'administration de gaz (20) à un patient (P) comprenant :
- un appareil de délivrance de NO (1) pour fournir un gaz à base de NO selon l'une des revendications 1 à 9, tel un mélange NO/N₂,
- un ventilateur médical (2) pour fournir un gaz à base d'oxygène, tel de l'air ou un mélange O₂/N₂,
- un circuit patient (3) auquel sont reliés fluidiquement l'appareil de délivrance de NO (1) et le ventilateur médical (2),
- et un capteur de débit proximal (35) agencés sur le circuit patient (3) et relié électriquement au ventilateur médical (2).

## Patentansprüche

1. Eine Vorrichtung zur Abgabe von NO (1), umfassend:
- einen Hauptgaskreislauf zum Leiten eines NO-haltigen Gases, insbesondere eines NO/N2-Gemisches,
- eine Analysenleitung (110), die Mittel zur Messung von NO/NO2 (120, 121) umfasst, die dafür konfiguriert sind, Messungen der Konzentration von NO und/oder NO2 innerhalb der besagten Analysenleitung (110) durchzuführen, und
- Steuerungsmittel (15), die mit den besagten Mitteln zur Messung von NO/NO2 (120, 121) verbunden und dafür konfiguriert sind, die von den besagten Mitteln zur Messung von NO/NO2 (120, 121) durchgeführten Messungen der Konzentration von NO und/oder NO2 zu verarbeiten,
**dadurch gekennzeichnet, dass**:
- die Analysenleitung (110) piezoelektrische Ansaugmittel (141) umfasst, die von den Steuerungsmitteln (15) gesteuert werden, um Gas mit einem gegebenen Ansaugdurchfluss anzusaugen,
- die Steuerungsmittel (15) ferner dafür konfiguriert sind, Mittel zur Erzeugung eines elektrischen Feldes zu steuern, die dafür konfiguriert sind, ein elektrisches Feld mit einer Anregungsfrequenz von mindestens etwa 20 kHz zu erzeugen,
- die piezoelektrischen Ansaugmittel (141) eine piezoelektrische Pumpe umfassen, die mindestens ein piezoelektrisches Material umfasst, das aus Materialien ausgewählt ist, die in der Lage sind, sich proportional zu einem an das besagte piezoelektrische Material angelegten elektrischen Feld zu verformen, und
- das besagte piezoelektrische Material dem besagten elektrischen Feld ausgesetzt wird, das von den Mitteln zur Erzeugung eines elektrischen Feldes erzeugt wird, so dass es sich unter der Wirkung des angelegten elektrischen Feldes ausdehnt oder zusammenzieht, wodurch ein Phänomen des Ansaugens oder Ausstoßens von Gas durch die piezoelektrische Pumpe erzeugt wird.

2. Vorrichtung nach Anspruch 1, **dadurch gekennzeichnet, dass** das piezoelektrische Material eine kristalline oder keramische Struktur aufweist.

3. Vorrichtung nach Anspruch 1, **dadurch gekennzeichnet, dass** der gegebene Ansaugdurchfluss geringer als 400 ml/min ist, vorzugsweise zwischen 200 und 300 ml/min.

4. Vorrichtung nach einem der Ansprüche 1 oder 2, **dadurch gekennzeichnet, dass** die Steuerungsmittel (15) dafür konfiguriert sind, Mittel zur Erzeugung eines elektrischen Feldes zu steuern, die dafür konfiguriert sind, ein elektrisches Feld mit einer Anregungsfrequenz zwischen 20 und 30 kHz zu erzeugen.

5. Vorrichtung nach Anspruch 1, **dadurch gekennzeichnet, dass** die Mittel zur Messung von NO/NO2 (120, 121) einen NO-Sensor (121) und einen NO2-Sensor (120) umfassen.

6. Vorrichtung nach Anspruch 1, **dadurch gekennzeichnet, dass** das piezoelektrische Material mindestens ein Metall umfasst.

7. Vorrichtung nach Anspruch 6, **dadurch gekennzeichnet, dass** das piezoelektrische Material Bleizirkonattitanat (PZT) umfasst.

8. Vorrichtung nach Anspruch 1, **dadurch gekennzeichnet, dass** die piezoelektrischen Ansaugmittel (141) eine piezoelektrische Saugpumpe umfassen, die während ihres Betriebs den dynamischen piezoelektrischen Effekt nutzt, der aus der Frequenzanregung resultiert.

9. Vorrichtung nach einem der Ansprüche 1, 6 oder 7, **dadurch gekennzeichnet, dass** das besagte mindestens eine piezoelektrische Material schichtweise auf einem metallischen Substrat abgeschieden ist, um eine piezoelektrische Membran mit einer Oberfläche von mindestens 30 mm² zu bilden.

10. Vorrichtung nach Anspruch 1, **dadurch gekennzeichnet, dass** die Mittel zur Erzeugung eines elektrischen Feldes einen elektrischen Stromwandler umfassen.

11. Vorrichtung nach Anspruch 10, **dadurch gekennzeichnet, dass** der elektrische Stromwandler mit elektrischem Gleichstrom versorgt wird.

12. Vorrichtung nach Anspruch 11, **dadurch gekennzeichnet, dass** der Stromwandler dafür konfiguriert ist, den Gleichstrom in Wechselstrom mit einer gegebenen Frequenz umzuwandeln.

13. Vorrichtung nach den Ansprüchen 1 und 12, **dadurch gekennzeichnet, dass** die Steuerungsmittel den Wert der Frequenz der Variation des Wechselstroms steuern.

14. Vorrichtung nach Anspruch 9, **dadurch gekennzeichnet, dass** die piezoelektrische Membran mit elektrischem Wechselstrom versorgt wird.

15. Eine Anlage zur Verabreichung von Gas (20) an einen Patienten (P), umfassend:
- eine Vorrichtung zur Abgabe von NO (1) zur Bereitstellung eines Gases auf NO-Basis nach einem der Ansprüche 1 bis 9, wie z.B. ein NO/N2-Gemisch,
- ein medizinisches Beatmungsgerät (2) zur Bereitstellung eines Gases auf Sauerstoffbasis, wie z.B. Luft oder ein O2/N2-Gemisch,
- einen Patientenkreislauf (3), an den die Vorrichtung zur Abgabe von NO (1) und das medizinische Beatmungsgerät (2) fluidisch angeschlossen sind,
- und einen proximalen Durchflusssensor (35), der am Patientenkreislauf (3) angeordnet und elektrisch mit dem medizinischen Beatmungsgerät (2) verbunden ist.

## Claims

1. An apparatus for delivering NO (1) comprising:
- a main gas circuit for conveying a gas containing NO, in particular an NO/N2 mixture,
- an analysis line (110) comprising means for measuring NO/NO2 (120, 121) configured to perform measurements of concentration of NO and/or NO2 within said analysis line (110), and
- control means (15) connected to said means for measuring NO/NO2 (120, 121) and configured to process the measurements of concentration of NO and/or NO2 performed by said means for measuring NO/NO2 (120, 121),
**characterized in that**:
- the analysis line (110) comprises piezoelectric suction means (141) controlled by the control means (15) to aspirate gas at a given suction flow rate,
- the control means (15) are further configured to control electric field generation means configured to generate an electric field at an excitation frequency of at least about 20 kHz,
- the piezoelectric suction means (141) comprise a piezoelectric pump comprising at least one piezoelectric material selected from materials capable of deforming proportionally to an electric field applied to said piezoelectric material, and
- said piezoelectric material is subjected to said electric field generated by the electric field generation means so as to expand or contract under the effect of the applied electric field, thereby generating a phenomenon of suction or expulsion of gas by the piezoelectric pump.

2. The apparatus according to claim 1, **characterized in that** the piezoelectric material has a crystalline or ceramic structure.

3. The apparatus according to claim 1, **characterized in that** the given suction flow rate is less than 400 mL/min, preferably between 200 and 300 mL/min.

4. The apparatus according to one of claims 1 or 2, **characterized in that** the control means (15) are configured to control electric field generation means configured to generate an electric field at an excitation frequency between 20 and 30 kHz.

5. The apparatus according to claim 1, **characterized in that** the means for measuring NO/NO2 (120, 121) comprise an NO sensor (121) and an NO2 sensor (120).

6. The apparatus according to claim 1, **characterized in that** the piezoelectric material comprises at least one metal.

7. The apparatus according to claim 6, **characterized in that** the piezoelectric material comprises lead zirconate titanate (PZT).

8. The apparatus according to claim 1, **characterized in that** the piezoelectric suction means (141) comprise a piezoelectric suction pump operating, during its operation, with the dynamic piezoelectric effect resulting from the frequentional excitation.

9. The apparatus according to one of claims 1, 6 or 7, **characterized in that** said at least one piezoelectric material is deposited as a layer on a metal substrate to form a piezoelectric membrane having a surface area of at least 30 mm².

10. The apparatus according to claim 1, **characterized in that** the electric field generation means comprise an electric current converter.

11. The apparatus according to claim 10, **characterized in that** the electric current converter is supplied with direct electric current.

12. The apparatus according to claim 11, **characterized in that** the current converter is configured to convert the direct current into alternating current at a given frequency.

13. The apparatus according to claims 1 and 12, **characterized in that** the control means control the value of the frequency of variation of the alternating current.

14. The apparatus according to claim 9, **characterized in that** the piezoelectric membrane is supplied with alternating electric current.

15. An installation for administering gas (20) to a patient (P) comprising:
- an apparatus for delivering NO (1) for supplying a gas based on NO according to any one of claims 1 to 9, such as an NO/N2 mixture,
- a medical ventilator (2) for supplying an oxygen-based gas, such as air or an O2/N2 mixture,
- a patient circuit (3) to which the NO delivery apparatus (1) and the medical ventilator (2) are fluidly connected,
- and a proximal flow sensor (35) arranged on the patient circuit (3) and electrically connected to the medical ventilator (2).
